# EUROPÄISCHE PATENTSCHRIFT

(11) **EP 0 901 011 B1**
(45) Veröffentlichungstag und Bekanntmachung des Hinweises auf die Patenterteilung: **29.03.2006**
(21) Anmeldenummer: 98112134.6
(22) Anmeldetag: 01.07.1998
(51) Int. Cl.: G01N 27/00

(54) **Oelalterungssensor**
Oil aging sensor
Capteur de viellisement d'huille

(30) Priorität: 29.08.1997 DE 19737714
(43) Veröffentlichungstag der Anmeldung: 10.03.1999
(73) Patentinhaber: Volkswagen Aktiengesellschaft, 38436 Wolfsburg (DE)
(72) Erfinder: Göpel, Wolfgang, Prof. Dr., 72016 Tübingen (DE)

(56) Entgegenhaltungen:
- EP-A- 0 442 314
- EP-A- 0 584 557
- EP-A- 0 597 452
- US-A- 4 560 534
- US-A- 4 638 286
- US-A- 5 023 133
- US-A- 5 312 762

## Beschreibung

Die Erfindung betrifft einen Ölqualitätssensor gemäß dem Oberbegriff des Anspruchs 1 und ein Verfahren gemäß dem Oberbegriff des Anspruchs 12.

Motorenöle haben sich im Laufe der Zeit zu High-Tech-Produkten entwickelt, die mit Voraussetzung sind, hohe Motorleistungen zur ermöglichen. "Motorenöle" stellen dabei eine Sammelbezeichnung für Grundölkomponenten aus Mineralöl, Hydrocrackanten und synthetischen Komponenten dar. Motorenöle enthalten noch Additive, die in der Regel als Fertigmischung ("Paket") zugegeben werden, sowie Viskositätsindex-Verbesserer (V1). Die Motorenöle dienen dabei als Schmierstoffe für die Motoren sowie als Kühl- und Abdichtmedium. Weiterhin sollen sie sämtliche Motorenteile reinigen und reinhalten. Die VI-Verbesserer bewirken ein günstigeres Viskositäts-Temperaturverhalten, als es die reinen Grundöle aufweisen. Der Anteil an Additiven und VI-Verbesserer liegt je nach Anforderungen, die an das Öl gestellt werden, üblicherweise zwischen 5 und 25 %.

Weitere Aufgaben der Additive in den Motorenölen sind die Verbesserung der Korrosionsschutzeigenschaften der Öle sowie die Verhinderung von Schlammablagerungen und Öleindickungen sowie der Verschleißschutz an den Reibpartnem unter allen auftretenden Belastungen. Die thermischen Beanspruchungen der Motorenöle sind hoch und liegen dabei im Ölsumpf durchschnittlich bei ca. 100 bis 150 °C. Im Bereich der oberen Kolbenringzone können Temperaturspitzen zwischen 200 und 350 °C auftreten.

Im Laufe ihres Gebrauchs altern die Öle, wobei in erster Linie die Additivkomponenten und die VI-Verbesserer abgebaut (verbraucht) werden. Einen erheblichen Anteil an der Ölalterung haben unverbrauchte, teilweise oxidierte und polymerisierte Kraftstoffkomponenten. Somit wird die Ölalterung durch Temperatureinwirkung und reaktionsfähige Verbrennungsprodukte (Radikale) sowie über das Überschreiten der Dispergierfähigkeit der Öle für Feststoffe und Alterungsprodukte hervorgerufen. Hierdurch werden dann die notwendigen Eigenschaften der Öle zum störungsfreien Betrieb der Motoren mitunter drastisch verschlechtert. Eine erhöhte Viskosität hat z. B. beim Startvorgang einen verlängerten Transport des Öles zu den Schmierstellen zur Folge, wodurch ein Anstieg des Verschleißes eintritt.

Der Verbrauch der Detergent- und Dispergieradditive hat eine Verschlechterung der Fähigkeit der Öle zur Reinhaltung der Motoren, insbesondere an kritischen Schmierstellen, zur Folge.

Wünschenswert ist es daher, die während des motorischen Betriebes zwangsläufig auftretende Verschlechterung der Eigenschaften der Motorenöle kontinuierlich oder in kurzen Zeitabschnitten, d. h. beispielsweise ein oder mehrmals während eines Betriebes einer Brennkraftmaschine, zu erfassen.

Bis heute ist es jedoch noch nicht gelungen, zuverlässige Meßfühler für eine Ölzustandsanalyse zu entwickeln, wobei für einen längeren Betrieb des Motorenöls im Motor, insbesondere bei instationären Motoren, eine on-board-Analyse, d. h. eine Analyse direkt am Motor, erforderlich ist.

Bisher wurden die unterschiedlichsten Ölsensoren entwickelt, die insbesondere die Viskosität, TAN (Total Acid Number) oder den Füllstand sensieren. Eine besondere Schwierigkeit ist hierbei die Verwendung unterschiedlicher Öle an ein und derselben Brennkraftmaschine sowie das Kompensieren unterschiedlicher Alterungseinflüsse auf die sensierte Eigenschaft. So ist es beispielsweise aus US-A 4,675,662 und 4,721,874, EP 527 176 B und JPN. Appl. Phys., 1993, Acoustic Plate Viskosity Sensor, bekannt, die sich mit der Alterung des Öls geänderte Viskosität als Meßgröße für den Ölzustand heranzuziehen. Dies geschieht über akustische Laufzeitveränderungen, die Phasenverschiebung oder über Eigenfrequenzänderungen eines Schwingquarzes. Problematisch sind hierbei zum einen die häufig fehlende Möglichkeit, die Messung on-board vorzunehmen, und zum anderen die möglichen gegenläufigen Effekte "Abbau des Motorenöls und Verdünnung durch Kraftstoff", die die Viskosität erniedrigen, gegenüber der "Verknüpfung der Abbauprodukte", die die Viskosität erhöhen, solange sie nicht als Schlamm ausfallen.

Die herkömmliche TAN- oder auch TBN- (Total Basic Number-)Analytik ist vom Grundprinzip für eine on-board-Messung nicht geeignet, da hierbei das Altöl mit KOH titriert wird. Neuere Ansätze, wie beispielsweise aus SAE 910497, US-A 4,675,662, 4,792,791 und 5,200,027 bekannt, zeigen hier interessante Lösungen, die beispielsweise mit Kapazitätssensoren, Messung der Ionenwanderung oder einer Potentialdifferenz, mit elektrochemischen Festkörperzellen und mit Korrosionssensoren arbeiten. Diese prinzipiell denkbaren Lösungsansätze zeigen jedoch nicht die geforderte Spezifität, sind teils noch zu groß und zu schwer oder benötigen ein Opferbauteil, was grundsätzlich unerwünscht ist.

Aus der US-PS 5,435,170 ist ein Ölqualitätssensor bekannt, bei dem als Sensorelement ein leitfähiges Ionenaustauscherharz eingesetzt wird. In einem unpolaren Lösungsmittel zeigt das Ionenaustauscherharz eine verhältnismäßig hohe Leitfähigkeit, die in Gegenwart von ionischen Gruppen, wie sie bei der Alterung eines Motorenöls auftreten, abnimmt. Für den Einsatz in dem Sensor werden die unterschiedlichsten Ionenaustauscherharze beschrieben, die als kleine Perlen in einen Leitfähigkeits-Meßkäfig gepackt werden. Wegen der relativ geringen Empfindlichkeit der Ionenaustauscherharze und der fehlenden Langzeitstabilität der Anordnung wird eine verhältnismäßig große Menge von 500 mg des Ionenaustauscherharzes in den Sensor eingesetzt.

Ferner sind noch indirekte Bestimmungsmethoden über mathematische Modelle (SAE 870403) und HC-Abgassensoren (DE 42 35 225) bekannt, die bislang auch noch nicht zu einem Durchbruch geführt haben. Auch Füllstandssensoren sind wenig geeignet, da diese beispielsweise bei einer starken Verdünnung des Motorenöls durch Kraftstoffe versagen.

Aufgabe der vorliegenden Erfindung ist es, einen Sensor zur Verfügung zu stellen, der auch on-board an einer Brennkraftmaschine Messungen vornehmen kann, wobei der Sensor weitgehend zuverlässig und störungsfrei die Alterung eines Öls bestimmen soll.

Bei dem eingangs beschriebenen Sensor wird die Erfindung gelöst mit den kennzeichnenden Merkmalen des Anspruchs 1.

Eine weitere Lösung der Erfindung zeigt das Verfahren gemäß dem Anspruch 11.

Die Unteransprüche zeigen bevorzugte Ausführungsformen, mit denen sich besonders günstige Sensoren bzw. Verwendungen von Sensoren durchführen lassen.

Um spezifische Wechselwirkungen zwischen TBN-bestimmenden Molekülen (oder TANbestimmenden Molekülen; wenn im folgenden nur auf TBN Bezug genommen wird, so gilt der Bezug gleichermaßen auch für TAN - total acid number -, sofern sich aus dem Inhalt nichts anderes ergibt) und der Oberfläche eines Sensors mit interdigitaler Elektrodenanordnung zu erzielen, ist die Oberfläche entsprechend zu modifizieren. Erfindungsgemäß wird die Oberfläche vorteilhaft mit einem temperaturbeständigen, ölunlöslichen Polymer beschichtet, welches mit den TBN-relevanten Verbindungen (insbesondere den basischen Verbindungen selbst) in Wechselwirkung tritt. Diese Wechselwirkungen führen zu mindestens einer Veränderung hinsichtlich der Masse, der Leitfähigkeit, der Kapazität, des Temperaturverhaltens, der Impedanz oder optischer Eigenschaften, die für die Auswertung der Wechselwirkungen herangezogen werden können.

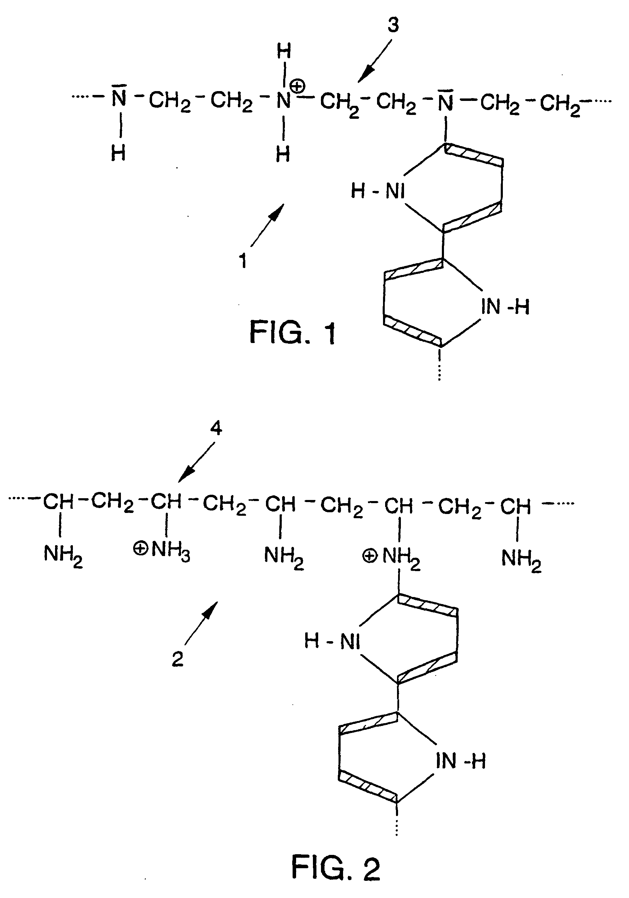

Bei den basischen, den TBN-bestimmenden, Molekülen handelt es sich im wesentlichen um anionische, organische substituierte Verbindungen vom Typ Phenolat, Salicylat, Phosphonat, Thiopyrophosphonat, Thiophosphonat, Sulfonat und Carbonat. Zur Erfassung dieser Moleküle wird vorteilhaft ein Polymer mit reversibler Adsorption dieser anorganischen Verbindungen eingesetzt. Vorzugsweise hat das Polymer nur schwache adsorbierende Eigenschaften gegenüber diesen Molekülen, um im Betrieb in einem Motorenöl die Reversibilität der Wechselwirkungen mit dem Polymer zu gewährleisten. Vorteilhaft hat das Polymer auch eine elektrische (auch ionische) Leitfähigkeit, wobei für eine einfache Bestimmung des Adsorptionsgrades der TBN-bestimmenden Moleküle eine adsorptionsgradabhängige Leitfähigkeit gemessen wird. Dabei können sowohl Gleichspannungs- als auch Wechselspannungsmessungen durchgeführt werden. Vorteilhaft können hierzu protonierte, polypyrrolsubstituierte Polyethylenimine (Fig.1) oder Polyvinylamine (Fig. 2) eingesetzt werden. Beide Polymere sind in der Lage, Anionen reversibel auszutauschen sowie Protonen und Elektronen zu leiten. Die elektrische Leitfähigkeit dieser Polymere ist außerdem vom Protonierungsgrad abhängig. Die im protonierten Polymer enthaltenen Gegenionen (beispielsweise Chlorid) können z. B. gegen die im gealterten Motorenöl enthaltenen alkylsubstituierten Sulfonate ausgetauscht werden.

Prinzipiell ist erfindungsgemäß der Sensor in allen Flüssigkeiten einsetzbar, in denen eine stoffliche Veränderung der Zusammensetzung erfolgt, d. h. in denen mindestens eine Komponente zu- bzw. abnimmt. Bevorzugt wird der Sensor zur Charakterisierung einer komplexen Flüssigkeit eingesetzt, d. h. in einer Flüssigkeit, die mehrere von der Struktur und Menge her unbekannte Komponenten enthält, wobei insbesondere eine Wiederholbarkeit der exakten Zusammensetzung der Flüssigkeit meist nicht gegeben ist, z. B. weil die Zusammensetzung der Flüssigkeit durch eine (nicht überschaubare) Vielfalt von Einflüssen bedingt ist. Ganz besonders vorteilhaft kommt der Sensor in einer ölhaltigen Flüssigkeit zum Einsatz, bevorzugt in einer Flüssigkeit, die mindestens zu 30 % und insbesondere zu mindestens 50 % ein Öl enthält. Neben dem Öl können noch verschiedene andere Komponenten vorliegen, die ggf. auch von dem Sensor erkannt werden können. Der Sensor kann ausgelegt sein auf einen oder mehrere Bestandteile des Öls, der/die im Laufe des Gebrauchs des Öls (bzw. der Flüssigkeit) abnehmen; vorteilhaft ist jedoch auch eine Auslegung des Sensors auf eine Zunahme eines oder mehrerer Bestandteile der Flüssigkeit. Vorteilhaft ist der Sensor ausgelegt auf eine oder mehrere Hauptkomponenten der Flüssigkeit, d. h. solche Komponenten, die den Hauptgewichtsanteil oder Hauptmengenanteil der Flüssigkeit, beispielsweise der Ölzusammensetzung, ausmachen.

Im Unterschied zu den eingangs beschriebenen Sensoren hat der erfindungsgemäße Sensor ein Sensormaterial, das kovalent an ein Substrat gebunden ist. Das Substrat dient der Fixierung des sensitiven Materials und ist entsprechend selbst vorteilhaft fest in einen Ölkreislauf einsetzbar bzw. eingesetzt. Hierzu ist das Substrat, an das das Sensormaterial gebunden ist, vorteilhaft an einem Befestigungsmittel festgelegt. Hierdurch wird vom Sensormaterial bis hin zum Befestigungselement eine materialschlüssige, formschlüssige und/oder kraftschlüssige Verbindung aller Materialien miteinander erreicht. Als Substrat kommen insbesondere mineralische oder metallische Stoffe, wie beispielsweise Keramiken, Oxide, Quarze, elektrische Kontakte, Edelmetalle etc., in Betracht. Vorteilhaft ist das Substrat ohne sensitive Eigenschaften und dient insbesondere der Signaltransduktion. Hierzu ist das Substrat beispielsweise als Schwingquarz, Kondensator, optischer oder elektrischer Leiter ausgebildet.

Weiterhin enthalten die erfindungsgemäßen Sensoren alternativ oder zusätzlich in der sensitiven Schicht einen polypyrrol-haltigen Ionenaustauscher. Hierbei kann das Polypyrrol selbst die Ionenaustausch-Eigenschaften haben, besonders vorteilhaft liegt jedoch ein Copolymer, beispielsweise mit Polyethylenimin oder Polyvinylamin vor. Das Polypyrrol kann selbst substituiert sein, wie auch das Copolymer. Durch die Kombination von Polypyrrol mit einem Copolymer erhält man einerseits günstige und für den Anwendungszweck optimal einstellbare Ionenaustauscheigenschaften und andererseits über das Polypyrrol eine elektrische Leitfähigkeit, die entsprechend der Beladung des Ionenaustauschers variiert. Gegenüber dem in der US 5,435,170 beschriebenen lonenaustauscher hat der erfindungsgemäße Einsatz an Polypyrrol die oben beschriebene Möglichkeit eines besseren und einfacheren Polymerdesigns.

Gegenüber bekannten Sensoren, wie z. B. Glaselektroden, unterscheidet sich der erfindungsgemäß eingesetzte Sensor durch mindestens eines der folgenden Merkmale:
- Der Bestandteil der Flüssigkeit hat ein Molekulargewicht ≥ 200;
- der Bestandteil der Flüssigkeit wird am Sensor im wesentlichen adsorbiert;
- es wird nicht ein Grenzflächenpotential, elektrochemisches Potential und/oder Wasserstoffpotential als charakteristische Größe bestimmt.

Der Bestandteil ist dabei so gewählt, daß er sich im Laufe des Gebrauchs der Flüssigkeit hinsichtlich seiner Affinität zu der sensitiven Schicht verändert, üblicherweise wird dies erreicht durch eine Konzentrationsänderung des Stoffes in der Flüssigkeit durch den Gebrauch derselben.

Erfindungsgemäß kann vorteilhaft ein (bzw. mehrere) Bestandteil der Flüssigkeit dadurch gemessen werden, daß der Bestandteil sich an die sensitive Schicht anlagert bzw. sich von dieser ablöst, wobei eine Gewichtsveränderung und/oder eine Veränderung der Leitfähigkeit der sensitiven Schicht stattfindet. Durch Messung der Veränderung erhält man ein Maß für die Anlagerung des/der fraglichen Bestandteils/e und hierdurch ein Maß für dessen/deren Konzentration. Zusätzlich oder alternativ wird gemäß der vorliegenden Erfindung ein (bzw. mehrere) Analyt (ein Bestandteil) in einem Öl und/oder ein (bzw. mehrere) öliger Analyt durch die Anlagerung des (bzw. der) Analyten in die sensitive Schicht bestimmt.

Als Analyt und/oder Öl eignet sich insbesondere ein Motorenöl, wie es beispielsweise in Brennkraftmaschinen zum Einsatz kommt. Vorteilhaft ist der Analyt hierbei zumindest ein Bestandteil eines neuen Motorenöls, er kann jedoch auch ein Bestandteil des bei der Benutzung des Motorenöls sich bildenden Altöls sein. Bevorzugt ist der Analyt hierbei wiederum ein aliphatischer Rest, wie er üblicherweise in mineralischen und/oder synthetischen Motorenölen vorliegt. Solche Kohlenwasserstoffreste haben üblicherweise ein Molekulargewicht zwischen 300 und 3.000. Durch den erfindungsgemäßen Einsatz der adaptierten sensitiven Schicht wird erreicht, daß sich beispielsweise weniger die neuen Ölkomponenten an die Schicht anlagern, sondern die Abbauprodukte (mit Keto-, Aldehyd- und/oder Säureresten bzw. deren Additions- und/oder Kondensationsprodukte).

Der Aufbau der sensitiven Schicht des Sensors aus einem Polymer ermöglicht eine einfache und kostengünstige Herstellung und leichte Adaption an einen Analyten. Als Polymer eignen sich insbesondere Polypyrrole, insbesondere modifizierte Polypyrrole, beispielsweise solche, die mit Polyethylenimin, Polyvinylamin oder sonstigen Ionenaustauschern verknüpft (Copolymere) sind.

Weiterhin wird erfindungsgemäß der Sensor vorteilhaft zusammen mit einem nichtsensitiven Sensor (Meßelement gleichen Aufbaus wie der Sensor, jedoch ohne die sensitiven Eigenschaften für einen Bestandteil der Flüssigkeit oder abgekapselt in einer Flüssigkeit konstanter Zusammensetzung wie Neuöl oder Altöl) eingesetzt, der als Referenz dient. Dies ermöglicht einen einfachen und sicheren Meßaufbau. Die Messung selbst erfolgt beispielsweise durch Versetzen der Schicht in Schwingungen, auch eine dielektrische Auswirkung an der Schicht oder insbesondere frequenzabhängige Leitfähigkeitsveränderung können als Meßprinzip herangezogen werden. Bei dem Versetzen der Schicht in Schwingungen wird die Schicht vorteilhaft auf einen Schwingkristall (Quarzkristall) aufgebracht, der dann zu Schwingungen angeregt wird. Durch die Beladung der sensitiven Schicht mit dem Analyt wird die Schicht entsprechend der Beladungsmenge schwerer, wodurch sich das Schwingungsverhalten verändert. Hieraus kann wiederum auf die Beladungsmenge zurückgeschlossen werden.

Zusätzlich zu der Referenz, über die - je nach Meßprinzip - auch die absolute Viskosität (bzw. eine Viskositätsveränderung) ermittelt werden kann, kommt vorteilhaft noch ein Temperatursensor zum Einsatz, da die Viskosität (insbesondere bei Motoröl) stark temperaturabhängig ist.

Das temperaturabhängige Viskositätsverhalten der flüssigen Phase kann hierbei beispielsweise in einem Kennfeld abgelegt sein, über das dann die Temperaturkorrektur der Meßwerte erfolgt. Alternativ oder zusätzlich kann die Messung auch nur bei einer oder mehreren festgelegten Temperaturen durchgeführt werden.

Vorteilhaft können mehrere Sensoren, insbesondere auf einem Substrat, parallel eingesetzt werden, die auf unterschiedliche Bestandteile der flüssigen Phase und/oder unterschiedliche flüssige Phasen abgestimmt sind. Hierdurch lassen sich unterschiedliche Veränderungen der flüssigen Phase erkennen. Dies ist insbesondere beim Einsatz des Sensors in einem Motoröl von Bedeutung, da hier zum einen unterschiedliche Öle mit unterschiedlichen Bestandteilen eingesetzt werden können, und zum anderen - je nach den Betriebsbedingungen des Motoröls - unterschiedliche Alterungsprozesse ablaufen können.

Zum Einsatz des Sensors gehört femer eine Auswerteelektronik, über die der Sensor einerseits betrieben und andererseits das Sensorsignal zu der gewünschten Information umgewandelt wird. Bei Motoröl kann die Information insbesondere ein Hinweis auf die Fälligkeit des Motorölwechsels sein.

Grundsätzlich kann für die vorliegende Erfindung praktisch jede Schicht eingesetzt werden, die an einen zu bestimmenden Bestandteil der Flüssigkeit (Analyt) angepaßt (adaptiert) ist, d. h. den Analyt entsprechend seiner Konzentration einlagert (bei einer hohen Konzentration) bzw. auslagert (bei einer niedrigen Konzentration des Analyten in der Flüssigkeit).

Die Erfindung wird im folgenden anhand von Zeichnungen und Ausführungsbeispielen näher beschrieben.

Es zeigen
- Figur 1: polypyrrolsubstituiertes Polyethylenimin;
- Figur 2: polypyrrolsubstituiertes Polyvinylamin;
- Figur 3: ein erstes Ankopplungsreagenz;
- Figur 4: ein zweites Ankopplungsreagenz;
- Figur 5: eine Sensoroberfläche und
- Figur 6: ein auf einen Sensor fixiertes Austauscherpolymer.

Die in den Figuren 1 und 2 dargestellten Copolymere 1 und 2 vereinigen zwei Eigenschaften in sich. Zum einen bilden sie über das Stickstoffatom in der Polyethylenimin- 3 bzw. Polyvinylaminkette 4 durch Quarternierung (Protonenanlagerung) des Stickstoffatoms einen Ionenaustauscher, zum anderen leiten sie Protonen und Elektronen. Zudem ist die elektrische Leitfähigkeit dieser Polymere abhängig vom Protonierungsgrad und dem Grad der Substitution des Gegenions, beispielsweise Chlorid, gegen beispielsweise alkylsubstituierte Sulfonate, wie sie mit der Alterung eines Motorenöls vermehrt vorliegen.

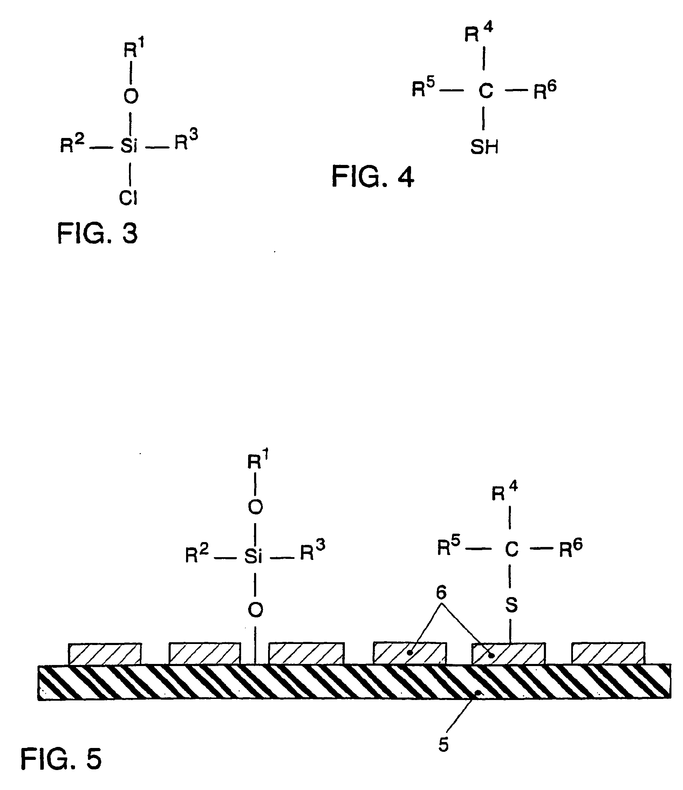

Für eine sichere On-Board-Diagnose der Ölalterung ist es erforderlich, daß die Sensoren langzeitstabil sind. Hierzu wird erfindungsgemäß das Polymer kovalent auf die Substratoberfläche des Sensors gebunden, wobei die Bindung einerseits auf oxydische Substrate 5 (beispielsweise Quarz) und andererseits auf Edelmetalle 6, wie Gold, erfolgt (dem Substrat fehlen die sensitiven Eigenschaften, es dient der Fixierung und/oder (elektrischen) Kontaktierung des sensitiven Materials (Polymer)). Hierzu sind die Ankopplungs-Reagenzien aus den Figuren 3 und 4 vorgesehen. Das alkylierte Silylfluorid aus Fig. 3 dient der Ankopplung auf mineralischem Substrat 5, wie beispielsweise Quarz; das alkylierte Thiol auf Fig. 4 ist zur Ankopplung auf metallischen Substraten 6 geeignet, wie beispielsweise Goldelektroden. Zur Ankopplung des Silylchlorids (Fig. 3) wird die Sensoroberfläche zuvor protoniert. Entsprechend angekoppelte Verankerungsreagenzien zeigt die Fig. 5. Zur Verbindung der Ankopplungsreagenzien mit den Polymeren 1 und 2 aus Fig. 1 und 2 dienen vorteilhaft aromatische Substituenten, wie beispielsweise Phenylgruppen. Hieraus ergibt sich beispielsweise folgender günstiger Aufbau für die Ankopplungs-Reagenzien aus Fig. 3 und 4.

R¹ = CH₃

R⁴=H

R²=R³=R⁵=R⁸=C₆H₅

Die Phenylgruppen können mit den Aminogruppen der Polymere unter Verwendung von Formaldehyd als Kopplungsreagenz verknüpft werden. Die Angabe der Reste R ist nur beispielhaft und kann variiert und für einzelne Polymere optimiert werden.

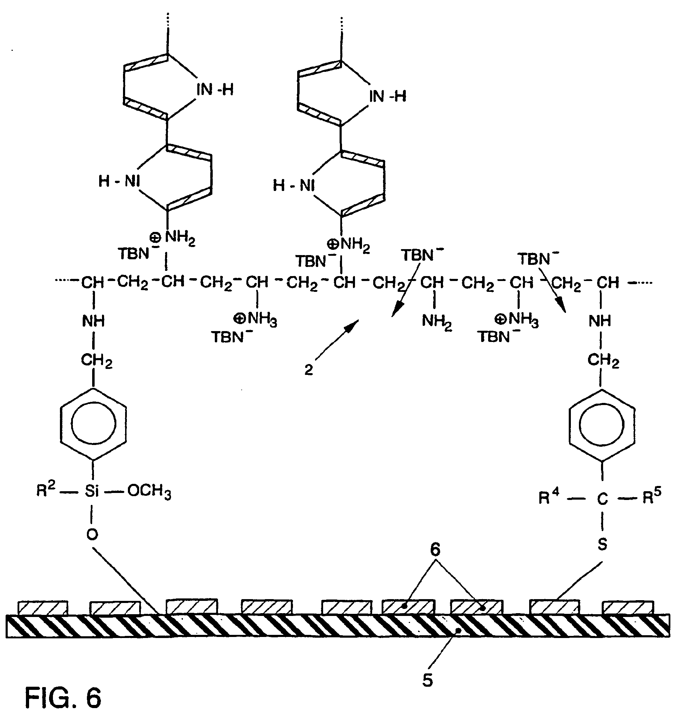

Der vollständige Sensoraufbau ist dann in Fig. 6 dargestellt. Das Ionenaustauscherpolymer 2 ist mit der Substratoberfläche und damit mit den Goldelektroden 6 verbunden, so daß über die Goldelektroden eine Widerstandsveränderung, die durch TBN-relevante anionische Moleküle hervorgerufen wird, gemessen werden kann. Vorteilhaft wird die Messung gegen eine Referenz durchgeführt, die beispielsweise ein gleichartiger Sensor, der sich stets in Neuöl befindet, oder eine elektronische Speicherung der relevanten Neuölwerte sein kann.

## Patentansprüche

1. Sensor mit einer sensitiven Schicht, die eine an mindestens einen Analyt adaptierte Oberfläche und/oder die ein an mindestens einen Analyt adaptiertes Schichtvolumen hat, die bzw. das entsprechend einer vorliegenden Affinität des Analyten zu der Schicht zur analytspezifischen erfassbaren wiederholten reversiblen Anlagerung des Analyten prädestiniert ist, wobei die sensitive Schicht aus einem Polymer aufgebaut und ein Ionenaustauscher ist, **dadurch gekennzeichnet, dass** die sensitive Schicht kovalent auf einem Substrat gebunden ist.

2. Sensor nach Anspruch 1, **dadurch gekennzeichnet, dass** die sensitive Schicht über ein Ankopplungsreagenz kovalent an das Substrat gebunden ist.

3. Sensor nach einem der Ansprüche 1 bis 3, **dadurch gekennzeichnet, dass** das Substrat mineralisch und/oder metallisch und/oder ein inerter (nicht sensitiver) Kunststoff ist.

4. Sensor nach Anspruch 4, **dadurch gekennzeichnet, dass** das Substrat ein Quarz ist.

5. Sensor nach Anspruch 4, **dadurch gekennzeichnet, dass** das Substrat ein elektrisch leitender Kontakt, insbesondere ein Edelmetallkontakt ist.

6. Sensor nach einem der Ansprüche 1 bis 6, **dadurch gekennzeichnet, dass** die Sensorschicht ein Anionenaustauscher ist.

7. Sensor nach einem der vorhergehenden Ansprüche, **dadurch gekennzeichnet, dass** die Schicht ein Copolymer ist.

8. Sensor nach einem der Ansprüche 5 bis 8, **dadurch gekennzeichnet, dass** die Schicht Polyethylenamin und/oder Polyvinylamin enthält.

9. Verwendung eines Sensors mit einer sensitiven Schicht gemäß einem der Ansprüche 1 bis 9 zur Erkennung einer Ölzusammensetzung, insbesondere einer Motorölzusammensetzung einer Brennkraftmaschine.

10. Verwendung nach Anspruch 10, **dadurch gekennzeichnet, dass** der Sensor zur Erkennung einer Alterung der Ölzusammensetzung verwendet wird.

11. Verfahren zur Erfassung der Anlagerung eines Analyten an einen Sensor gemäß einem der Ansprüche 1 bis 9, **dadurch gekennzeichnet, dass** die Veränderung der Masse, des frequenzabhängigen elektrischen Widerstands, der nichtlinearen elektrischen Eigenschaften, der thermischen Eigenschaften, der Impedanz und/oder der optischen Eigenschaften der sensitiven Schicht erfasst wird.

12. Verfahren nach Anspruch 12, **dadurch gekennzeichnet, dass** der Sensor mit einer Referenz eingesetzt wird.

13. Verfahren zur Herstellung eines Ölsensors, über den der Gebrauchszustand bzw. eine Alterung eines Öls, insbesondere ein Motoröl einer Brennkraftmaschine, ermittelbar ist, wobei der Sensor einen Ionenaustauscher enthält, **dadurch gekennzeichnet, dass** ein Polypyrrol enthaltender Ionenaustauscher kovalent an ein Substrat gebunden wird.

14. Verfahren nach Anspruch 14, **dadurch gekennzeichnet, dass** das Substrat mineralisch, insbesondere ein Quarz und/oder metallisch, insbesondere ein elektrisch leitender Kontakt, insbesondere Edelmetallkontakt, und/oder ein inerter (nicht sensitiver) Kunststoff ist.

15. Verfahren nach Anspruch 14 oder 15, **dadurch gekennzeichnet, dass** das Substrat vor der Verbindung mit dem Ionenaustauscher mit einer oder mehreren Ankopplungsreagenzien verbunden wird.

## Claims

1. Sensor having a sensitive layer which has a surface adapted to at least one analyte and/or which has a layer volume adapted to at least one analyte, said surface or said layer volume being preorganized in accordance with a certain affinity of the analyte to the layer for analyte-specific detectable repeated reversible adsorption of the analyte, the sensitive layer being composed of a polymer and being an ion exchanger, **characterized in that** the sensitive layer is bonded covalently to a substrate.

2. Sensor according to Claim 1, **characterized in that** the sensitive layer has been bonded to the substrate covalently by means of a coupling reagent.

3. Sensor according to either of Claims 1 and 2, **characterized in that** the substrate is mineral and/or metallic and/or an inert (insensitive) polymer.

4. Sensor according to Claim 3, **characterized in that** the substrate is a quartz.

5. Sensor according to Claim 3, **characterized in that** the substrate is an electrically conductive contact, especially a noble metal contact.

6. Sensor according to one of Claims 1 to 5, **characterized in that** the sensor layer is an anion exchanger.

7. Sensor according to one of the preceding claims, **characterized in that** the layer is a copolymer.

8. Sensor according to one of Claims 4 to 7, **characterized in that** the layer comprises polyethyleneamine and/or polyvinylamine.

9. Use of a sensor having a sensitive layer according to one of Claims 1 to 8 for recognizing an oil composition, especially a motor oil composition of an internal combustion engine.

10. Use according to Claim 9, **characterized in that** the sensor is used to recognize ageing of the oil composition.

11. Method for detecting the adsorption of an analyte onto a sensor according to one of Claims 1 to 8, **characterized in that** the change in the mass, in the frequency-dependent electrical resistance, in the nonlinear electrical properties, in the thermal properties, in the impedance and/or in the optical properties of the sensitive layer is detected.

12. Process according to Claim 11, **characterized in that** the sensor is used with a reference.

13. Process for producing an oil sensor, by means of which the state of use or ageing of an oil, especially a motor oil of an internal combustion engine, can be determined, the sensor comprising an ion exchanger, **characterized in that** an ion exchanger comprising polypyrrole is bonded covalently to a substrate.

14. Process according to Claim 13, **characterized in that** the substrate is mineral, especially a quartz, and/or metallic; especially an electrically conductive contact, especially noble metal contact, and/or an inert (insensitive) polymer.

15. Process according to Claim 13 or 14, **characterized in that** the substrate, before being bonded with the ion exchanger, is bonded with one or more coupling reagents.

## Revendications

1. Capteur muni d'une couche sensible qui possède une surface adaptée à au moins un analyte et/ou un volume de couche adapté à au moins un analyte, laquelle ou lequel est prédestiné à une réticulation réversible répétée détectable spécifique à l'analyte de l'analyte conformément à une affinité existante de l'analyte avec la couche, la couche sensible étant constituée d'un polymère et étant un échangeur d'ions, **caractérisé en ce que** la couche sensible est liée de manière covalente à un substrat.

2. Capteur selon la revendication 1, **caractérisé en ce que** la couche sensible est liée de manière covalente au substrat par le biais d'un agent réactif d'accouplement.

3. Capteur selon la revendication 1 ou 2, **caractérisé en ce que** le substrat est minéral et/ou métallique et/ou une matière plastique inerte (non sensible).

4. Capteur selon la revendication 3, **caractérisé en ce que** le substrat est un quartz.

5. Capteur selon la revendication 3, **caractérisé en ce que** le substrat est un contact conducteur d'électricité, notamment un contact en métal noble.

6. Capteur selon l'une des revendications 1 à 5, **caractérisé en ce que** la couche de détection est un échangeur d'anions.

7. Capteur selon l'une des revendications précédentes, **caractérisé en ce que** la couche est un copolymère.

8. Capteur selon l'une des revendications 4 à 7, **caractérisé en ce que** la couche contient du polyéthylénamine et/ou du polyvinylamine.

9. Utilisation d'un capteur muni d'une couche sensible selon l'une des revendications 1 à 8 pour détecter la composition d'une huile, notamment la composition d'une huile moteur d'un moteur à combustion interne.

10. Utilisation selon la revendication 9, **caractérisée en ce que** le capteur est utilisé pour détecter un vieillissement de la composition de l'huile.

11. Procédé pour détecter la réticulation d'un analyte selon l'une des revendications 1 à 8, **caractérisé en ce que** la modification de la masse, de la résistance électrique en fonction de la fréquence, des propriétés électriques non linéaires, des propriétés thermiques, de l'impédance et/ou des propriétés optiques de la couche sensible est détectée.

12. Procédé selon la revendication 11, **caractérisé en ce que** le capteur est utilisé avec une référence.

13. Procédé pour fabriquer un capteur d'huile à l'aide duquel peut être déterminé l'état d'usure ou un vieillissement d'une huile, notamment d'une huile moteur d'un moteur à combustion interne, le capteur contenant un échangeur d'ions, **caractérisé en ce qu'**un échangeur d'ions contenant un polypyrrole est lié de manière covalente à un substrat.

14. Procédé selon la revendication 13, **caractérisé en ce que** le substrat est minéral, notamment un quartz et/ou métallique, notamment un contact conducteur d'électricité, notamment un contact en métal noble, et/ou une matière plastique inerte (non sensible).

15. Procédé selon la revendication 13 ou 14, **caractérisé en ce que** le substrat est lié avec un ou plusieurs agents réactifs d'accouplement avant la liaison avec l'échangeur d'ions.
